(19) Europäisches Patentamt
European Patent Office
Office européen des brevets

(11) Veröffentlichungsnummer : **0 089 538**
**B1**

(12) # EUROPÄISCHE PATENTSCHRIFT

(45) Veröffentlichungstag der Patentschrift : **30.01.85**

(51) Int. Cl.[4] : **C 07 C 79/46**, C 07 C 76/00

(21) Anmeldenummer : **83102201.7**

(22) Anmeldetag : **07.03.83**

(54) **Optisch aktive Phenoxybenzoesäure-Derivate, Verfahren zu ihrer Herstellung und ihre Verwendung als Herbizide.**

(30) Priorität : **19.03.82 DE 3210055**

(43) Veröffentlichungstag der Anmeldung : **28.09.83 Patentblatt 83/39**

(45) Bekanntmachung des Hinweises auf die Patenterteilung : **30.01.85 Patentblatt 85/05**

(84) Benannte Vertragsstaaten : **AT BE CH DE FR GB IT LI NL**

(56) Entgegenhaltungen : **GB-A- 2 058 055**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Cölln, Reimer, Dr.**
**In den Birken 67**
**D-5600 Wuppertal 1 (DE)**
Erfinder : **Eue, Ludwig, Dr.**
**Paul-Klee-Strasse 36**
**D-5090 Leverkusen 1 (DE)**
Erfinder : **Schmidt, Robert R., Dr.**
**Im Waldwinkel 110**
**D-5060 Bergisch-Gladbach 2 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 089 538 B1

Jouve, 18, rue St-Denis, 75001 Paris, France

**Beschreibung**

Die Erfindung betrifft neue rechtsdrehende Enantiomere*) von Phenoxybenzoesäure-Derivaten, ein Verfahren zu ihrer Herstellung und ihre Verwendung als Herbizide.

Es ist bereits bekannt, daß Racemate der Verbindungen 5-(2-Chlor-4-trifluormethyl-phenoxy)-2-nitro-benzoesäure-(1-ethoxycarbonylethyl)-ester und 5-(2,6-Dichlor-4-trifluormethyl-phenoxy)-2-nitro-benzoesäure-(1-ethoxy-carbonyl-ethyl)-ester zur Unkrautbekämpfung eingesetzt werden können (vgl. DE-OS 29 50 401 und DE-OS 30 29 728). Die Wirksamkeit dieser Stoffe ist gut, jedoch sind die selektiven herbiziden Eigenschaften nicht immer voll befriedigend.

Es wurden jetzt die neuen rechtsdrehenden Enantiomeren der Phenoxybenzoesäure-Derivate der Formel

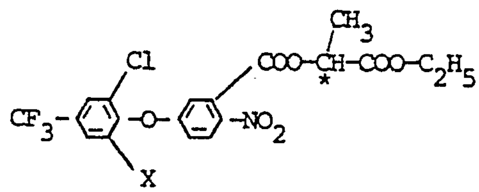

(I)

in welcher

X für Wasserstoff oder Chlor steht,

gefunden.

Weiterhin wurde gefunden, daß man die neuen rechtsdrehenden Enantiomeren der Phenoxybenzoesäure-Derivate der Formel (I) erhält, wenn man Phenoxybenzoesäurechloride der Formel

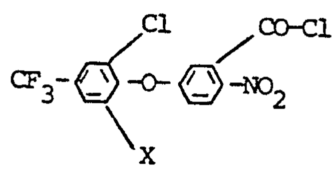

(II)

in welcher

X die oben angegebene Bedeutung hat,

mit dem linksdrehenden Enantiomeren des Milchsäure-ethylesters der Formel

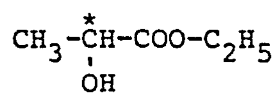

(III)

gegebenenfalls in Gegenwart eines Säureakzeptors sowie gegebenenfalls in Gegenwart eines Verdünnungsmittels umsetzt.

Schließlich wurde gefunden, daß sich die neuen rechtsdrehenden Enantiomeren der Phenoxybenzoesäure-Derivate der Formel (I) durch eine hervorragende herbizide Wirksamkeit auszeichnen.

Überraschenderweise besitzen die neuen rechtsdrehenden Enantiomeren der Phenoxybenzoesäure-Derivate der Formel (I) wesentlich bessere herbizide Eigenschaften als die entsprechenden racemischen Verbindungen, die aus dem Stand der Technik als hoch wirksame Herbizide bekannt sind.

Die erfindungsgemäßen Verbindungen sind durch die Formel (I) definiert. In dieser Formel ist das asymmetrische Kohlenstoffatom durch ein (*) gekennzeichnet.

Verwendet man bei dem erfindungsgemäßen Verfahren 5-(2,6-Dichlor-4-trifluormethyl-phenoxy)-2-nitrobenzoesäurechlorid und linksdrehendes Enantiomeres des Milchsäure-ethylesters, so kann der Verlauf der Umsetzung durch das folgende Formelschema wiedergegeben werden :

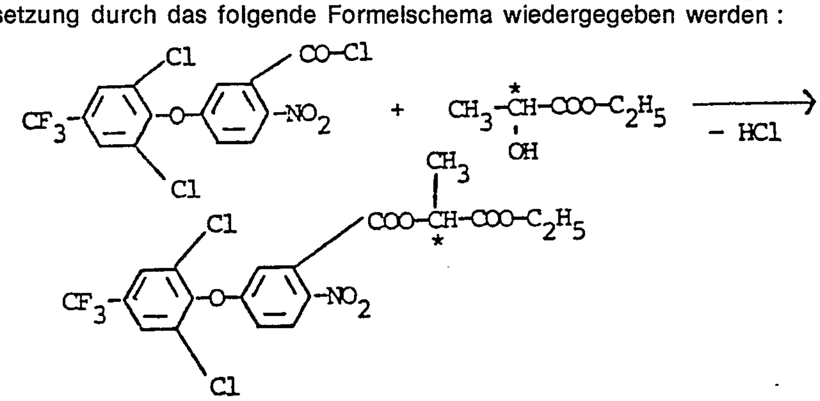


*) Unter rechtsdrehenden Enantiomeren sind hier jeweils diejenigen optisch aktiven Verbindungen zu verstehen, welche die Schwingungsebene von linear polarisiertem Licht nach rechts drehen. Diese Verbindungen besitzen am asymmetrisch substituierten Kohlenstoffatom im vorliegenden Fall die S-Konfiguration.

Die bei dem erfindungsgemäßen Verfahren als Ausgangsstoffe benötigten Phenoxybenzoesäurechloride sind durch die Formel (II) eindeutig definiert. In dieser Formel steht X für Wasserstoff oder Chlor.

Die Phenoxybenzoesäurechloride der Formel (II) sind bekannt (vgl. DE-OS 29 50 401).

Das bei dem erfindungsgemäßen Verfahren weiterhin als Ausgangsstoff benötigte linksdrehende Enantiomere des Milchsäure-ethylesters ist ebenfalls bekannt : Die Verbindung besitzt einen Drehwert von $[\alpha]_D^{20} = -12^o$ (neat) und einen Brechungsindex von $n_D^{20} = 1{,}413\,0$.

Das erfindungsgemäße Verfahren zur Herstellung der neuen rechtsdrehenden Enantiomeren der Phenoxybenzoesäure-Derivate der Formel (I) wird vorzugsweise unter Verwendung von Verdünnungsmitteln durchgeführt. Als derartige Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methyl-isopropyl-, und Methyl-isobutylketon, Ester, wie Essigsäuremethylester und -ethylester, Nitrile, wie z. B. Acetonitril und Propionitril, Amide, wie z. B. Dimethylformamid, Dimethylacetamid und N-Methyl-pyrrolidon, sowie Dimethylsulfoxid, Tetramethylensulfon und Hexamethylphosphorsäuretriamid.

Die erfindungsgemäße Umsetzung wird außerdem vorzugsweise in Gegenwart eines Säurebindemittels vorgenommen. Als Säureakzeptoren können dabei alle üblichen Säurebindemittel eingesetzt werden. Vorzugsweise in Frage kommen Alkali-hydroxide, wie z. B. Natrium- und Kaliumhydroxid, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin.

Die Reaktionstemperatur kann innerhalb eines größeren Bereiches variiert werden. Im allgemeinen arbeitet man zwischen − 20 und + 100 °C, vorzugsweise zwischen 0 und + 50 °C.

Das erfindungsgemäße Verfahren wird im allgemeinen bei Normaldruck durchgeführt.

Zur Durchführung des erfindungsgemäßen Verfahrens werden die Ausgangsstoffe im allgemeinen in angenähert äquimolaren Mengen eingesetzt. Die Umsetzung wird im allgemeinen in einem geeigneten Verdünnungsmittel in Gegenwart eines Säureakzeptors durchgeführt, und das Reaktionsgemisch wird mehrere Stunden bei der erforderlichen Temperatur gerührt. Die anschließende Aufarbeitung erfolgt nach üblichen Methoden.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden :

Dikotyle Unkräuter der Gattungen : Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen : Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen : Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen : Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen übergeführt werden, wie Lösungen, Emulsionen, Spritzpulver, Suspensionen, Pulver, Stäubemittel, lösliche Pulver, Granulate, Suspensions-Emulsionskonzentrate, Wirkstoffimprägnierte Natur- und synthetische Stoffe, Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln, und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage :

Aromaten, wie Xylol, Toluol, oder Alkylnaphthaline, chlorierte Aromaten oder chlorierte aliphatische Kohlenwasserstoffe, wie Chlorbenzol, Chlorethylen oder Methylenchlorid, aliphatische Kohlenwasserstoffe, wie Cyclohexan oder Paraffine, z. B. Erdölfraktionen, Alkohole, wie Butanol oder Glycol sowie deren Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyethylen-Fettsäure-Ester, Polyoxyethylen-Fettalkohol-Ether, z. B. Alkylarylpolyglykolether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azofarbstoffe und Metallphthalocyaninfarbstoffe und Spurennährstoffe wie Salze von Eisen, Mangan, Bor, Kupfer, Kobalt, Molybdän and Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden.

Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die Herstellung und die Verwendung der erfindungsgemäßen Stoffe geht aus den nachfolgenden Beispielen hervor.

## Herstellungsbeispiele

### Beispiel 1

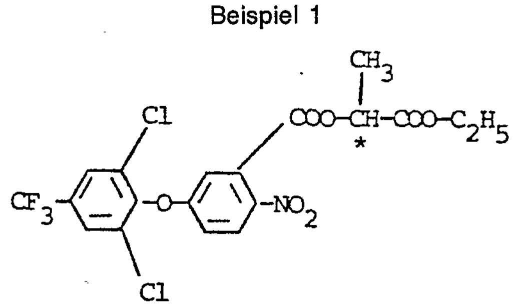

446,3 g (3,779 Mol) des linksdrehenden Enantiomeren des Milchsäureethylesters ($[\alpha]_D^{20}$ = – 12 °C (neat) ; $n_D^{20}$ = 1,413 0) werden zusammen mit 404 g (4 Mol) Triethylamin in 6 Litern Toluol vorgelegt. Bei 0-5 °C wird unter Außenkühlung und Rühren innerhalb von 30 Minuten eine Lösung von 1 566 g

(3,778 Mol) 5-(2,6-Dichlor-4-trifluormethylphenoxy)-2-nitro-benzoesäurechlorid in 3 Litern Toluol zugegeben. Danach wird 16 Stunden bei 20-25 °C nachgerührt und dann aufgearbeitet. Dazu versetzt man das Reaktionsgemisch mit 5 Litern gesättigter, wäßriger Kochsalz-Lösung und trennt die Phasen. Die organische Phase wird nacheinander mit einem Gemisch aus 5 Litern gesättigter, wäßriger Kochsalz-Lösung und 1 Liter Salzsäure, dann mit 5 Litern 5 %iger wäßriger Natriumhydrogencarbonat-Lösung und schließlich mit 5 Litern Waser gewaschen. Danach wird die organische Phase bei einer Badtemperatur von 50 °C unter vermindertem Druck eingedampft. Der verbleibende Rückstand wird durch einstündiges Erhitzen auf 50 bis 60 °C im Hochvakuum von den letzten flüchtigen Anteilen befreit. Man erhält auf diese Weise 1 720 g (98,6 % der Theorie) an rechtsdrehendem Enantiomeren des 5-(2,6-Dichlor-4-trifluormethyl-phenoxy)-2-nitrobenzoesäure-(1-ethoxycarbonyl-ethyl)-esters.

Schmelzpunkt : 53 °C
Drehwert : $[\alpha]_D^{20} = + 16{,}1°$ (1-molare Lösung in Chloroform ; Küvettenlänge = 10 cm)

Beispiel 2

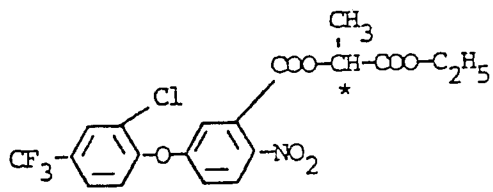

828 g (7 Mol) des linksdrehenden Enantiomeren des Milchsäure-ethylesters $[\alpha]_D^{20} = - 12°$ (neat) ; $n_D^{20} = 1{,}413\,0$) werden zusammen mit 778 g (7,7 Mol) Triethylamin in 11 Litern Toluol vorgelegt. Bei 0-5 °C wird unter Außenkühlung und Rühren innerhalb von 35 Minuten eine Lösung von 2 660 g (7 Mol) 5-(2-Chlor-4-trifluormethylphenoxy)-2-nitro-benzoesäurechlorid in 4,5 Litern Toluol zugegeben. Danach wird 16 Stunden bei 20-25 °C nachgerührt und dann aufgearbeitet. Dazu versetzt man das Reaktionsgemisch mit verdünnter, wäßriger Salzsäure und trennt die Phasen. Die organische Phase wird zweimal mit Wasser gewaschen und nach dem Trocknen über Natriumsulfat unter vermindertem Druck eingedampft. Der verbleibende Rückstand wird durch einstündiges Erhitzen auf 50 bis 60 °C im Hochvakuum von den letzten flüchtigen Anteilen befreit. Man erhält auf diese Weise 2 740 g (85 % der Theorie) an rechtsdrehendem Enantiomeren des 5-(2-Chlor-4-trifluormethyl-phenoxy)-2-nitro-benzoesäure-(1-ethoxycarbonyl-ethyl)-esters.

Drehwert : $[\alpha]_D^{20} = + 11{,}5°$ (1-molare Lösung in Chloroform ; Küvettenlänge = 10 cm)

In den nachstehend beschriebenen Tests werden die folgenden Stoffe eingesetzt :

(A) = 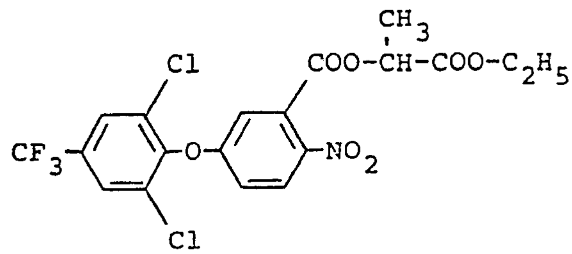 (racemisch)

(B) = 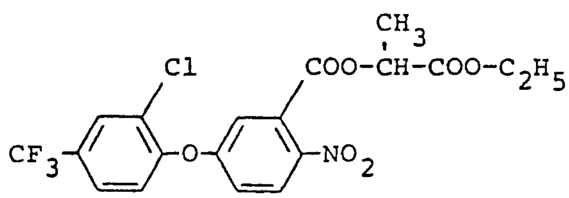 (racemisch)

(1) = 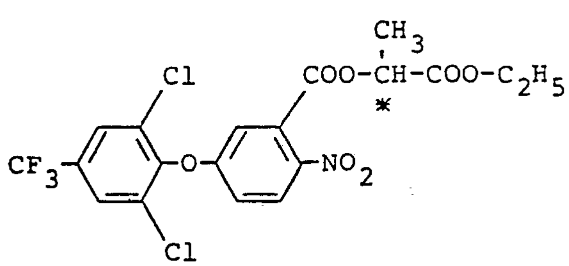 (rechtsdrehend)

(2) = 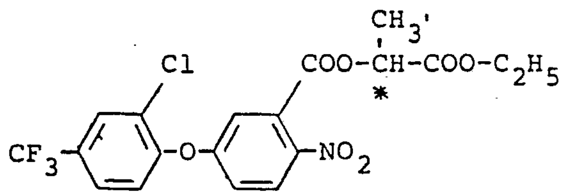 (rechtsdrehend)

Beispiel A

Pre-emergence-Test

Lösungsmittel : 5 Gewichtsteile Aceton
Emulgator : 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung

In diesem Test zeigen die erfindungsgemäßen Wirkstoffe gemäß Beispielen 1 und 2 eine bessere herbizide Wirksamkeit als die entsprechenden racemischen Verbindungen (A) und (B).

(Siehe Tabelle A, Seite 7 f.)

Tabelle A

Pre-emergence-Test

| Wirkstoff | Wirkstoff-aufwand (kg/ha) | Wirkung (in %) Dicotyledoneae *) | Monocotyledoneae **) | Rüben | Soja | Mais |
|---|---|---|---|---|---|---|
| (A) (bekannt) | 1 | 95,6 | 69,3 | 100 | 40 | 10 |
| (B) (bekannt) | 1 | 81,4 | 61,4 | 100 | 0 | 0 |
| (1) (erfindungsgemäß) | 0,45 | 88,6 | 42,1 | 99 | 10 | 0 |
| (2) (erfindungsgemäß) | 0,51 | 77,1 | 74,1 | 99 | 0 | 0 |

*) = durchschnittliche Wirkung bei mehreren Dicotylen
**) = durchschnittliche Wirkung bei mehreren Monocotylen

Beispiel B

Post-emergence-Test

Lösungsmittel : 5 Gewichtsteile Aceton
Emulgator : 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, daß die jeweils gewünschten Wirkstoffmengen pro Flächeneinheit ausgebracht werden. Die Konzentration der Spritzbrühe wird so gewählt, daß in 2 000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung

In diesem Test zeigen die erfindungsgemäßen Wirkstoffe gemäß Beispielen 1 und 2 eine bessere herbizide Wirksamkeit als die entsprechenden racemischen Verbindungen (A) und (B).

(Siehe Tabelle B, Seite 9 f.)

Tabelle B

Post-emergence-Test

| Wirkstoff | Wirkstoff-aufwand (kg/ha) | W i r k u n g (in %) | | | | |
|---|---|---|---|---|---|---|
| | | Dicotyledoneae *) | Monocotyledoneae **) | Rüben | Soja | Mais |
| (A) (bekannt) | 1 | 100 | 92 | 100 | 100 | 95 |
| (B) (bekannt) | 1 | 99,9 | 89,1 | 100 | 99 | 95 |
| (1) (erfindungsgemäß) | 0,45 | 99,9 | 89,9 | 100 | 100 | 95 |
| (2) (erfindungsgemäß) | 0,51 | 99,9 | 83,3 | 100 | 99 | 100 |

*) = durchschnittliche Wirkung bei mehreren Dicotylen
**) = durchschnittliche Wirkung bei mehreren Monocotylen.

**Ansprüche**

1. Rechtsdrehende Enantiomere der Phenoxybenzoesäure-Derivate der Formel

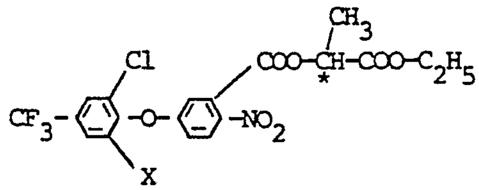

(I)

in welcher
X für Wasserstoff oder Chlor steht.

2. Verfahren zur Herstellung der rechtsdrehenden Enantiomeren der Phenoxybenzoesäure-Derivate der Formel

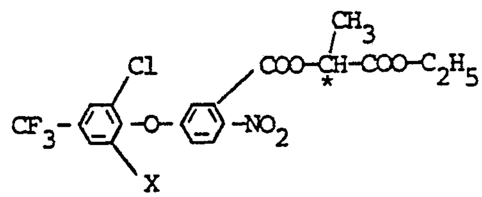

(I)

in welcher
X für Wasserstoff oder Chlor steht,
dadurch gekennzeichnet, daß man Phenoxybenzoesäure-chloride der Formel

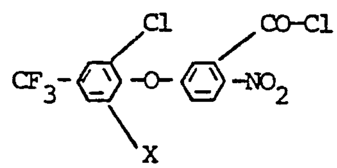

(II)

in welcher
X die oben angegebene Bedeutung hat,
mit dem linksdrehenden Enantiomeren des Milchsäureethylesters der Formel

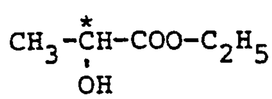

(III)

gegebenenfalls in Gegenwart eines Säureakzeptors sowie gegebenenfalls in Gegenwart eines Verdünnungsmittels umsetzt.

3. Herbizide Mittel, gekennzeichnet durch einen Gehalt an mindestens einem rechtsdrehenden Enantiomeren eines Phenoxybenzoesäure-Derivates der Formel (I).

4. Verwendung von rechtsdrehenden Enantiomeren der Phenoxybenzoesäure-Derivate der Formel (I) als Herbizide.

5. Verfahren zur Bekämpfung von Unkräutern, dadurch gekennzeichnet, daß man rechtsdrehende Enantiomere der Phenoxybenzoesäure-Derivate der Formel (I) auf die Unkräuter und/oder deren Lebensraum ausbringt.

6. Verfahren zur Herstellung von herbiziden Mitteln, dadurch gekennzeichnet, daß man rechtsdrehende Enantiomere der Phenoxybenzoesäure-Derivate der Formel (I) mit Streckmitteln und/oder oberflächenaktiven Stoffen vermischt.

**Claims**

1. Dextrorotatory enantiomers of phenoxybenzoic acid derivatives of the formula

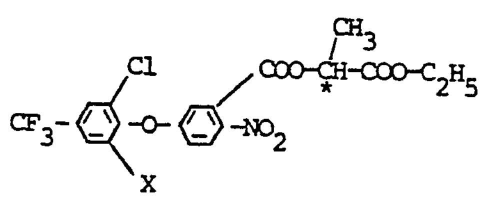

(I)

in which

X represents hydrogen or chlorine.

2. Process for the preparation of the dextrorotatory enantiomers of the phenoxybenzoic acid derivatives of the formula

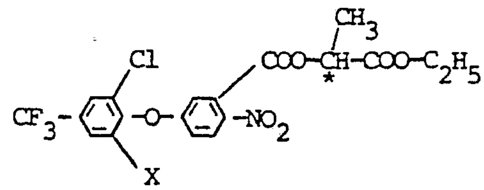

(I)

in which

X represents hydrogen or chlorine,

characterised in that phenoxybenzoic acid-chlorides of the formula

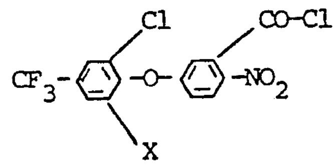

(II)

in which

X has the meaning given above,

are reacted with the laevorotatory enantiomer of ethyl lactate of the formula

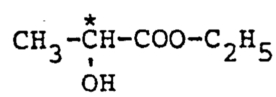

(III)

if appropriate in the presence of an acid acceptor and if appropriate in the presence of a diluent.

3. Herbicidal agents, characterised in that they contain at least one dextrorotatory enantiomer of a phenoxybenzoic acid derivative of the formula (I).

4. Use, as herbicides, of dextrorotatory enantiomers of phenoxybenzoic acid derivatives of the formula (I).

5. Process for combating weeds, characterised in that dextrorotatory enantiomers of phenoxybenzoic acid derivatives of the formula (I) are applied to the weeds and/or their habitat.

6. Process for the preparation of herbicidal agents, characterised in that dextrorotatory enantiomers of phenoxybenzoic acid derivatives of the formula (I) are mixed with extenders and/or surface-active substances.

**Revendications**

1. Enantiomères dextrogyres des dérivés de l'acide phénoxybenzoïque de formule

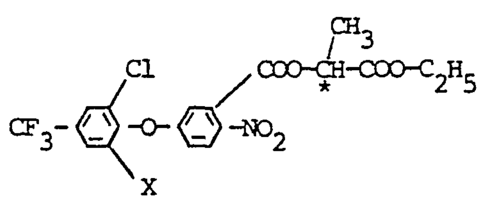

(I)

dans laquelle

X représente l'hydrogène ou le chlore.

2. Procédé de préparation des énantiomères dextrogyres des dérivés de l'acide phénoxybenzoïque de formule

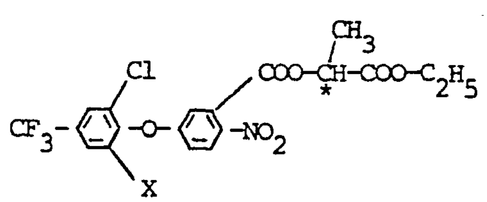

(I)

dans laquelle

X représente l'hydrogène ou le chlore,

procédé caractérisé en ce qu'on fait réagir des chlorures d'acides phénoxybenzoïques de formule

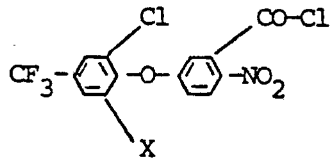

(II)

dans laquelle

X a le sens indiqué ci-dessus,

avec les énantiomères lévogyres du lactate d'éthyle, de formule

$$CH_3-\overset{*}{C}H(OH)-COO-C_2H_5$$

(III)

en opérant éventuellement en présence d'un accepteur d'acide et éventuellement aussi en présence d'un diluant.

3. Herbicide, caractérisé en ce qu'il contient au moins un énantiomère dextrogyre d'un dérivé d'acide phénoxybenzoïque de formule (I).

4. Utilisation comme herbicides d'énantiomères dextrogyres de dérivés de l'acide phénoxybenzoïque de formule (I).

5. Procédé pour combattre les mauvaises herbes, caractérisé en ce qu'on applique sur les mauvaises herbes et/ou sur leur biotope ou espace vital des énantiomères dextrogyres des dérivés de l'acide phénoxybenzoïque de formule (I).

6. Procédé de préparation d'herbicides, caractérisé en ce qu'on mélange des énantiomères dextrogyres de dérivés de l'acide phénoxybenzoïque de formule (I) avec des agents d'allongement et/ou des substances tensioactives.